# EUROPÄISCHE PATENTSCHRIFT

(11) **EP 0 528 287 B1**
(45) Veröffentlichungstag und Bekanntmachung des Hinweises auf die Patenterteilung: **11.11.1998**
(21) Anmeldenummer: 92113469.8
(22) Anmeldetag: 07.08.1992
(51) Int. Cl.: C07F 17/00, C08F 4/642

(54) **Verbrückte, chirale Metallocene, Verfahren zu ihrer Herstellung und ihre Verwendung als Katalysatoren**
Bridged chiral metallocenes, process for their preparation and their use as catalysts
Métallocènes pontés chirales, procédé pour leur préparation et leur utilisation comme catalyseurs

(30) Priorität: 08.08.1991 DE 4126234
(43) Veröffentlichungstag der Anmeldung: 24.02.1993
(73) Patentinhaber: TARGOR GmbH, 55116 Mainz (DE)
(72) Erfinder: Rohrmann, Jürgen, Dr., W-6233 Kelkheim am Taunus (DE)

(56) Entgegenhaltungen:
- EP-A- 320 762
- EP-A- 524 624
- EP-A- 0 185 918
- EP-A- 0 420 436
- EP-A- 0 423 101
- EP-A- 0 426 643

## Beschreibung

Die vorliegende Erfindung betrifft in erster Linie neue verbrückte, chirale Metallocene, die vorteilhaft als Katalysatorkomponenten bei der Herstellung von hochmolekularem Polyethylen oder von Polymeren höherer α-Olefine mit reduzierter Taktizität eingesetzt werden können.

Hochmolekulares Polyethylen eignet sich für die Herstellung von Hohlkörpern und Formteilen, während Polymere mit reduzierter Taktizität beispielsweise als Beschichtungsmaterial oder zur Herstellung von Dachbahnen verwendet werden können.

Verbrückte Metallocene sind in Kombination mit Aluminoxanen hochaktive stereospezifische Katalysatoren zur Herstellung von Polyolefinen (US 4,769,510). Die katalytischen Eigenschaften dieser Systeme und die Struktur der entstehenden Polymere werden im wesentlichen durch die Struktur des Ligandsystems am Zentralatom des Metallocens bestimmt. Hierbei spielt, neben elektronischen und sterischen Faktoren, die Symmetrie des Ligandsystems eine große Rolle (New. J. Chem. 102 (1990) 499).
Werden bei der Polymerisation von einem 1-Olefin wie Propylen achirale Metallocene eingesetzt, so entsteht ataktisches Polypropylen.
Werden bei der Polymerisation von Propylen chirale Metallocene mit C₂-symmetrischem Ligandsystem, wie verbrückte Bisindenylsysteme oder verbrückte, substituierte Biscyclopentadienylsysteme eingesetzt, so entsteht isotaktisches Polypropylen (EP-A 185 918).
Werden bei der Polymerisation von Propylen Metallocene mit C₂-symmetrischem Ligandsystem, wie verbrückte Cyclopentadienyl-Fluorenylsysteme, eingesetzt, so entsteht syndiotaktisches Polypropylen (EP-A 351 391).
Werden bei der Polymerisation von Propylen chirale Metallocene mit unsymmetrischem Ligandsystem, wie verbrückte Cyclopentadienyl-Indenylsysteme eingesetzt, so entstehen Isoblockstrukturen (DE-OS 38 26 075).

In EP-A- 0 423 101 werden Cyclopentadienyl/Fluorenyl-Metallocene beschrieben, die sich zur Herstellung von hemi-isotaktischen Polyolefinen eignen.

Es besteht somit ein großes Interesse daran, weitere Strukturvarianten von verbrückten Metallocenen als Katalysatcren zur Polymerisation von Olefinen einzusetzen.

Bekannt sind bisher verbrückte Metallocene, bei denen als π-Liganden Cyclopentadienyl/Cyclopentadienyl-, -, Cyclopentadienyl/lndenyl-, Cyclopentadienyl/Fluorenyl- und Indenyl/Indenyl-Kombinationen vorliegen sowie deren substituierte Derivate (J. Organomet. Chem. 288 (1985) 63, EP-A 316 155).

Bei der Synthese von Indenyl/Indenyl- und Cyclopentadienyl/Cyclopentadienyl-Komplexen fallen stets zwei konstitutionsisomere Formen an, eine racemische und eine meso-Form. Das Verhältnis der Isomeren beträgt in der Regel 1:1. Die Trennung dieser beiden Formen ist präparativ aufwendig und in vielen Fällen nicht oder nur unvollständig möglich. Außerdem wird die Ausbeute an der gewünschten racemischen Form dadurch stark gemindert.

Aus der nicht vorveröffentlichten Patentanmeldung EP-A- 0 524 624 sind bestimmte Indenyl/Fluorenyl-Metallocene bekannt.

Für die stereospezifische Polymerisation von 1-Olefinen müssen als Katalysatoren die reinen Racemate eingesetzt werden, da die meso-Formen unspezifisch polymerisieren.

Die Aufgabe, die mit der vorliegenden Erfindung gelost wird, bestand somit darin, eine neue Verbindungsklasse von chiralen Metallocenen zu finden, bei der auf die aufwendige Abtrennung der meso-Form verzichtet werden kann.

Die Erfindung betrifft somit die Verbindungen der Formel I 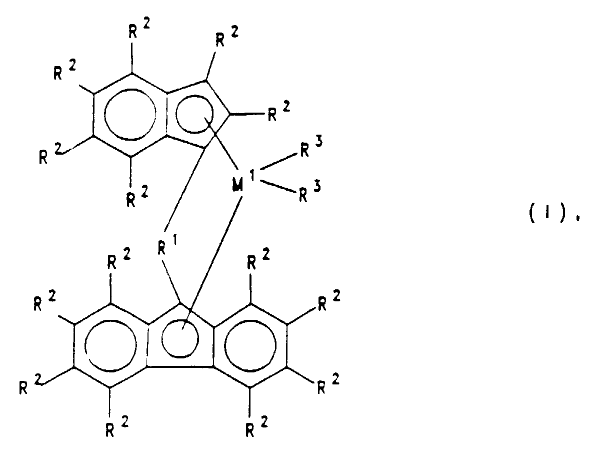 worin M¹ ein Metall aus der Gruppe Titan, Zirkon, Hafnium, Vanadin, Niob und Tantal ist,
R¹ 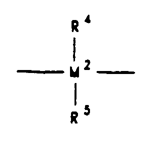 ist, wobei M² Kohlenstoff, Silizium, Germanium oder Zinn bedeutet,
R⁴ und R⁵ gleich oder verschieden sind und ein Wasserstoffatom, ein Halogenatom, eine C₁-C₁₀-Alkylgruppe, eine C₆-C₁₀-Arylgruppe, eine C₂-C₁₀-Alkenylgruppe, eine C₇-C₄₀-Arylalkylgruppe, eine C₈-C₄₀-Arylalkenylgruppe oder eine C₇-C₄₀-Alkylarylgruppe bedeuten, oder R⁴ und R⁵ zusammen mit dem sie verbindenden Atom einen Ring bilden ,
die Reste R² gleich oder verschieden sind und Wasserstoff, eine C₁-C₁₀-Alkylgruppe, eine C₆-C₁₀-Arylgruppe, eine C₂-C₁₀-Alkenylgruppe, eine C₇-C₄₀-Arylalkylgruppe, eine C₈-C₄₀-Arylalkenylgruppe, eine C₇-C₄₀-Alkylarylgruppe oder ein Halogenatom bedeuten, und
die Reste R³ gleich oder verschieden und eine C₁-C₁₀-Alkylgruppe, eine C₁-C₁₀-Alkoxygruppe, eine C₆-C₁₀-Arylgruppe, eine C₅-C₁₀-Aryloxygruppe, eine C₂-C₁₀-Alkenylgruppe, eine C₇-C₄₀-Arylalkylgruppe, eine C₈-C₄₀-Arylalkenylgruppe, eine C₇-C₄₀-Alkylarylgruppe, Wasserstoff oder ein Halogenatom bedeuten.

Dabei steht Alkyl für geradkettiges oder verzweigtes Alkyl und Halogen für Fluor, Chlor, Brom und Jod, insbesondere für Chlor.

In der Formel I gilt bevorzugt, daß M¹ Titan, Zirkon oder Hafnium bedeutet, M² für Kohlenstoff oder Silizium steht, R⁴ und R⁵ gleich sind und Wasserstoff oder eine C₁-C₄-Alkylgruppe bedeuten, R² Wasserstoff oder eine C₁-C₄-Alkylgruppe bedeutet, und die Reste R³ gleich sind und Halogen oder eine C₁-C₄-Alkylgruppe bedeuten.

Insbesondere gilt, daß M¹ Zirkon ist, M² für Kohlenstoff oder Silizium steht, R⁴ und R⁵ gleich sind und Methyl oder Ethyl bedeuten, R² Wasserstoff oder eine Methylgruppe bedeutet und die Reste R³ gleich sind und Chlor oder Methyl bedeuten.

Als bevorzugte Vertreter der erfindungsgemäßen Metallocene seien (Isopropyliden(9-fluorenyl)(1-indenyl))zirkondichlorid und (Dimethylsilyl(9-fluorenyl)(1-indenyl))zirkondichlorid genannt.

Die Metallocene I sind chiral und fallen bei der Synthese als Racemat an. Aus Symmetriegründen kann keine unerwünschte meso-Form ausgebildet werden. Bevorzugt wird das Racemat als Katalysatorkomponente bei der Olefinpolymerisation eingesetzt. Auch die reine R- oder S-Form kann als Polymerisationskatalysator verwendet werden. Die Trennung der Stereoisomeren ist im Prinzip bekannt.

Die vorliegende Erfindung betrifft ferner ein Verfahren zur Herstellung der Metallocene I, dadurch gekennzeichnet, daß man
a) eine Verbindung der Formel II 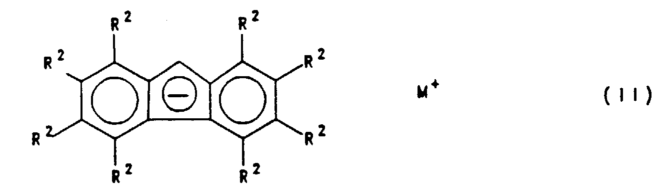 mit einer Verbindung der Formel III

   X-R¹-X (III)

   zu einer Verbindung der Formel IV 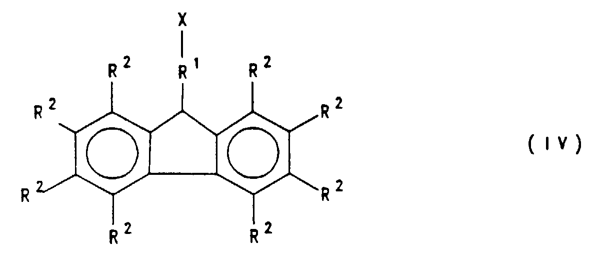 und diese mit einer Verbindung der Formel V 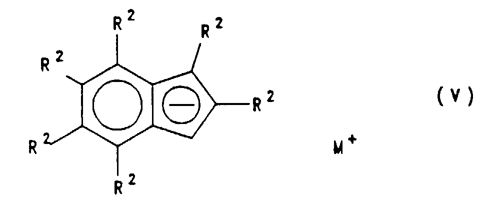 zu einer Verbindung der Formel VI 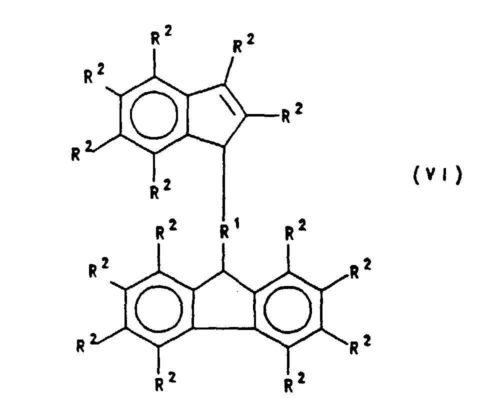 umsetzt, wobei R¹ und R² die für Formel I angegebenen Bedeutungen besitzen, X für eine nucleophile Abgangsgruppe wie Halogen oder O-Tosyl steht und M ein Alkalimetall bedeutet, oder
b) für Verbindungen der Formel I mit 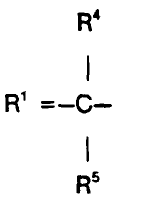 eine Verbindung der Formel VII 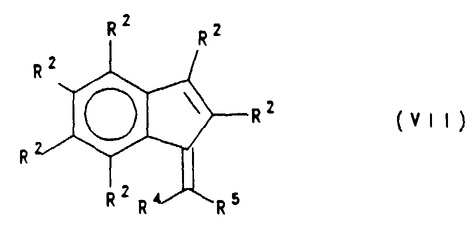 mit einer Verbindung der Formel II zu einer Verbindung der Formel VI umsetzt, wobei die Substituenten die für Formel I genannten Bedeutungen besitzen, und das unter a) oder b) erhaltene Reaktionsprodukt VI mit einer Verbindung der Formel VIII

   M¹X'₄ (VIII),

   worin X' für Halogen steht und M¹ die in Formel I genannte Bedeutung hat, umsetzt, und das so erhaltene Reaktionsprodukt gegebenenfalls derivatisiert.

Die Ligandsysteme der Formel VI können nach bekannten Methoden (J. Organomet. Chem. 288 (1985)63) zu den Metallocendichloriden und mit Alkylierungsmitteln, wie z. B. Lithiumalkylen, nach literaturbekannten Methoden (J. Amer. Chem. Soc. 95 (1973)6263) zu den Metallocenen der allgemeinen Formel (I) umgesetzt werden:

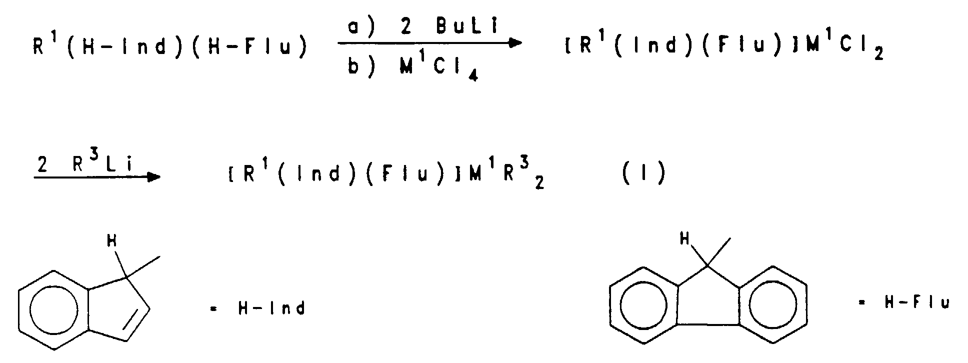

### Verfahrensvariante a):

Das Fluorenylanion II (Darstellung siehe Variante b)), das auch substituiert sein kann, wird in einem inerten Lösemittel (siehe Variante b)) mit einem Überschuß an Reagenz der Formel X-R¹-X umgesetzt, um bevorzugt eine Monosubstitution zu erzielen. Hierzu wird das Reagenz X-R¹-X vorgelegt und mit dem Fluorenylanion langsam versetzt, so daß ständig ein Überschuß an dem Brückenreagenz vorliegt. Als Abgangsgruppen X sind bevorzugt Halogenatome geeignet. Das Monosubstitutionsprodukt IV wird anschließend mit dem Indenylanion V, das auch substituiert sein kann, in einem inerten Lösemittel (siehe Variante b)) im molaren Verhältnis 0,5:1 bis 1:0,5, bevorzugt 1:1, umgesetzt. Hierbei kann das Monosubstitutionsprodukt oder die Indenylkomponente vorgelegt und mit der anderen Komponente versetzt werden, bevorzugt wird die Indenylkomponente langsam zum Monosubstitutionsprodukt gegeben. Die Reaktionstemperatur liegt im Bereich -78°C bis +120°C, bevorzugt im Bereich -40°C bis +40°C.

Das Indenylanion wird hergestellt durch Deprotonierung des entsprechenden Indens oder substituierten Indens (Herstellung siehe Variante b)) mit einer starken Base in den o.a. Lösemitteln. Geeignet sind Butyllithium, Natriumhydrid, Kaliumhydrid, Natriumamid oder elementares Natrium oder Kalium.

Nach hydrolytischer Aufarbeitung des Reaktionsansatzes können die Ligandsysteme der Formel VI durch Extraktion, Umkristallisation oder Säulenchromatographie isoliert und gereinigt werden.

Verfahrensvariante b) für Verbindungen I mit

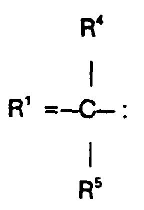

Die gemaß dem nachfolgenden Reaktionsschema als Ausgangsstoffe dienenden substituierten Indene sind kommerziell erhältlich oder können nach literaturbekannten Methoden durch Ankondensieren des Fünfringes an einen substituierten Aromaten hergestellt werden (Bull. Soc. Chim. Fr., 11(1973)3092; Bull. Soc. Chim. Fr., 3(1967)987; J. Org. Chem. 55(1990)247). Diese werden nach literaturbekannten Methoden durch Umsetzung mit Ketonen der u.a. Formel und Basen wie Natriumethanolat in Ethanol in substituierte Benzofulvene VII überführt (Annalen, 347(1906)257). Diese werden dann mit dem Anion des Fluorens, das auch substituiert sein kann, in einem inerten Lösemittel im molaren Verhältnis 0,5:1 bis 1:0,5, bevorzugt 1:1, zur Reaktion gebracht. Geeignete Lösemittel sind aliphatische oder aromatische Kohlenwasserstoffe oder Ether. Besonders geeignet sind Tetrahydrofuran, Diethylether und Toluol. Das Benzofulven oder die Fluorenylkomponente II kann vorgelegt und die andere Komponente zugegeben werden, bevorzugt wird das Benzofulven zur Fluorenylkomponente getropft. Die Reaktionstemperatur liegt im Bereich -78°C bis +120°C, bevorzugt im Bereich -40°C bis +40°C. Die Reaktionszeit beträgt 1 bis 100 h, bevorzugt 8 bis 24 h.

Das Fluorenylanion wird hergestellt durch Deprotonierung der kommerziell erhältlichen Fluoren-Derivate mit einer starken Base in den o.a. Lösemitteln. Geeignet sind Butyllithium, Natriumhydrid, Kaliumhydrid, Natriumamid oder elementares Natrium oder Kalium.

Nach hydrolytischer Aufarbeitung des Reaktionsansatzes können die Ligandsysteme der Formel VI durch Extraktion, Umkristallisation oder Säulenchromatographie isoliert und gereinigt werden.

Die Ligandsysteme der Formel VI können somit prinzipiell nach folgendem Reaktionsschema hergestellt werden:

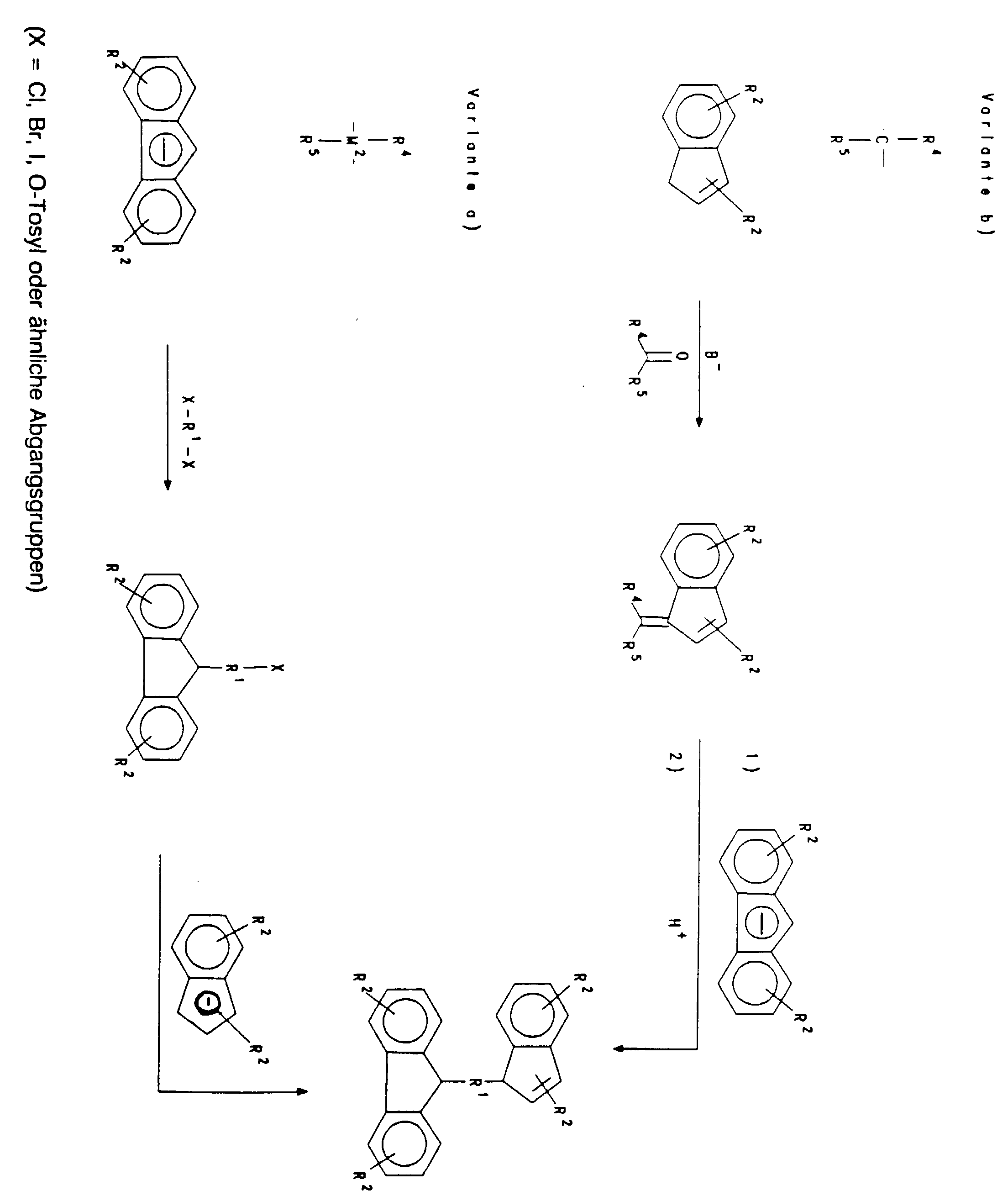

Die Metallocene der Formel I sind geeignete Katalysatorkomponenten für die Olefinpolymerisation.

Die vorliegende Erfindung betrifft somit auch die Verwendung der Metallocene I als Katalysatorkomponente bei der Olefinpolymerisation.

Bevorzugt wird als Cokatalysator bei der Olefinpolymerisation ein Aluminoxan der Formel (A) 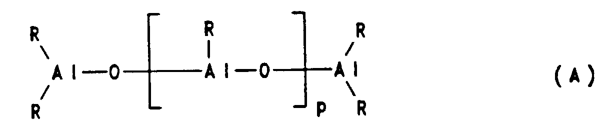 für den linearen Typ und/oder der Formel (B) 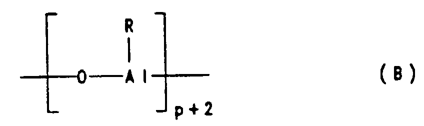 für den cyclischen Typ verwendet, wobei in den Formeln (A) und (B) die Reste R gleich oder verschieden sein können und eine C₁-C₆-Alkylgruppe, eine C₆-C₁₈-Arylgruppe oder Wasserstoff bedeuten, und p eine ganze Zahl von 2 bis 50, bevorzugt 10 bis 35 bedeutet.

Bevorzugt sind die Reste R gleich und bedeuten Methyl, Isobutyl, Phenyl oder Benzyl, besonders bevorzugt Methyl.

Sind die Reste R unterschiedlich, so sind sie bevorzugt Methyl und Wasserstoff oder alternativ Methyl und Isobutyl, wobei Wasserstoff bzw. Isobutyl bevorzugt zu 0,01 - 40 % (Zahl der Reste R) enthalten sind.

Das Aluminoxan kann auf verschiedene Arten nach bekannten Verfahren hergestellt werden. Eine der Methoden ist beispielsweise, daß eine Aluminiumkohlenwasserstoffverbindung und/oder eine Hydridoaluminiumkohlenwasserstoffverbindung mit Wasser (gasförmig, fest, flüssig oder gebunden - beispielsweise als Kristallwasser) in einem inerten Lösungsmittel (wie z.B. Toluol) umgesetzt wird. Zur Herstellung eines Aluminoxans mit verschiedenen Alkylgruppen R werden entsprechend der gewünschten Zusammensetzung zwei verschiedene Aluminiumtrialkyle (AlR₃ + AlR'₃) mit Wasser umgesetzt (vgl. S. Pasynkiewicz, Polyhedron 9(1990)429 und EP-A 302 424).

Die genaue Struktur der Aluminoxane A und B ist nicht bekannt.

Unabhängig von der Art der Herstellung ist allen Aluminoxanlösungen ein wechselnder Gehalt an nicht umgesetzter Aluminiumausgangsverbindung, die in freier Form oder als Addukt vorliegt, gemeinsam.

Es ist möglich, das Metallocen I vor dem Einsatz in der Polymerisationsreaktion mit einem Aluminoxan der Formel A und/oder B vorzuaktivieren. Dadurch wird die Polymerisationsaktivität deutlich erhöht und die Kornmorphologie verbessert.

Die Voraktivierung der Übergangsmetallverbindung wird in Lösung vorgenommen. Bevorzugt wird dabei das Metallocen in einer Lösung des Aluminoxans in einem inerten Kohlenwasserstoff aufgelöst. Als inerter Kohlenwasserstoff eignet sich ein aliphatischer oder aromatischer Kohlenwasserstoff. Bevorzugt wird Toluol verwendet.

Die Konzentration des Aluminoxans in der Lösung liegt im Bereich von ca. 1 Gew.-% bis zur Sättigungsgrenze, vorzugsweise von 5 bis 30 Gew.-%, jeweils bezogen auf die Gesamtlösung. Das Metallocen kann in der gleichen Konzentration eingesetzt werden, vorzugsweise wird es jedoch in einer Menge von 10⁻⁴ -1 mol pro mol Aluminoxan eingesetzt. Die Voraktivierungszeit beträgt 5 Minuten bis 60 Stunden, vorzugsweise 5 bis 60 Minuten. Man arbeitet bei einer Temperatur von -78°C bis 100°C, vorzugsweise 0 bis 70°C.

Das Metallocen kann auch vorpolymerisiert oder auf einen Träger aufgebracht werden. Zur Vorpolymerisation wird bevorzugt das (oder eines der) in der Polymerisation eingesetzte(n) Olefin(e) verwendet.

Geeignete Träger sind beispielsweise Silikagele, Aluminiumoxide, festes Aluminoxan oder andere anorganische Trägermaterialien. Ein geeignetes Trägermaterial ist auch ein Polyolefinpulver in feinverteilter Form.

Eine weitere mögliche Ausgestaltung des Verfahrens besteht darin, daß man an Stelle oder neben eines Aluminoxans eine salzartige Verbindung der Formel RₓNH₄₋ₓBR'₄ oder der Formel R₃PHBR'₄ als Cokatalysator verwendet. Dabei sind x = 1, 2 oder 3, R = Alkyl oder Aryl, gleich oder verschieden, und R' = Aryl, das auch fluoriert oder teilfluoriert sein kann. In diesem Fall besteht der Katalysator aus dem Reaktionsprodukt eines Metallocens mit einer der genannten Verbindungen (vgl. EP-A 277 004).

Die Polymerisation oder Copolymerisation wird in bekannter Weise in Lösung, in Suspension oder in der Gasphase, kontinuierlich oder diskontinuierlich, ein- oder mehrstufig bei einer Temperatur von 0 bis 150°C, vorzugsweise 30 bis 80°C, durchgeführt. Polymerisiert oder copolymerisiert werden Olefine der Formel R^{a}-CH=CH-R^{b}. In dieser Formel sind R^{a} und R^{b} gleich oder verschieden und bedeuten ein Wasserstoffatom oder einen Alkylrest mit 1 bis 14 C-Atomen.

Insbesondere wird Propylen und Ethylen polymerisiert.

Als Molmassenregler wird, falls erforderlich, Wasserstoff zugegeben. Der Gesamtdruck im Polymerisationssystem beträgt 0,5 bis 100 bar. Bevorzugt ist die Polymerisation in dem technisch besonders interessanten Druckbereich von 5 bis 64 bar.

Dabei wird das Metallocen in einer Konzentration, bezogen auf das Übergangsmetall, von 10⁻³ bis 10⁻⁸, vorzugweise 10⁻⁴ bis 10⁻⁷ mol Übergangsmetall pro dm³ Lösemittel bzw. pro dm³ Reaktorvolumen angewendet. Das Aluminoxan wird in einer Konzentration von 10⁻⁵ bis 10⁻¹ mol, vorzugsweise 10⁻⁴ bis 10⁻² mol pro dm³ Lösemittel bzw. pro dm³ Reaktorvolumen verwendet. Prinzipiell sind aber auch höhere Konzentrationen möglich.

Wenn die Polymerisation als Suspensions- oder Lösungspolymerisation durchgeführt wird, wird ein für das Ziegler-Niederdruckverfahren gebräuchliches inertes Lösemittel verwendet. Beispielsweise arbeitet man in einem aliphatischen oder cycloaliphatischen Kohlenwasserstoff; als solcher sei beispielsweise Butan, Pentan, Hexan, Heptan, Isooctan, Cyclohexan, Methylcyclohexan genannt.

Weiterhin kann eine Benzin- bzw. hydrierte Dieselölfraktion benutzt werden. Brauchbar ist auch Toluol. Bevorzugt wird im flüssigen Monomeren polymerisiert.

Werden inerte Lösemittel verwendet, werden die Monomeren gasförmig oder flüssig zudosiert.

Die Dauer der Polymerisation ist beliebig, da das erfindungsgemäß zu verwendende Katalysatorsystem einen nur geringen zeitabhängigen Abfall der Polymerisationsaktivität zeigt.

Die nachfolgenden Beispiele sollen die Erfindung näher erläutern.

Alle nachfolgenden Arbeitsoperationen wurden, soweit nicht anders angegeben, unter Ar-Atmosphäre durchgeführt (Schlenktechnik).

### Beispiel 1

### Isopropyliden(9-fluorenyl)-1-inden (1)

Eine Lösung von 10,2 g (60,2 mmol) Fluoren in 65 ml Tetrahydrofuran wurde bei Raumtemperatur mit 24,1 ml (60,2 mmol) einer 2,5 M Butyllithiumlösung in Hexan versetzt. Anschließend wurde noch 1 h unter Rückfluß erhitzt. 9,9 g (60,2 mmol) Benzofulven (Herstellung Annalen 347 (1906) 257) wurden in 50 ml Tetrahydrofuran gelöst und bei Raumtemperatur innerhalb 4 h zu der roten Lösung des Lithiumfluorens getropft. Die Lösung wurde anschließend 16 h bei Raumtemperatur gerührt und noch 2 h unter Rückfluß erhitzt. Die dunkelbraune Reaktionsmischung wurde auf Eis gegossen und mit Methylenchlorid extrahiert. Die vereinigten organischen Phasen wurden über Natriumsulfat getrocknet und zur Trockne eingeengt. Der Rückstand wurde an 550 g Kieselgel 60 mit einem Laufmittelgemisch aus Hexan/Methylenchlorid chromatographiert, wobei der Anteil an Methylenchlorid im Verlauf der Chromatographie von 3 % allmählich auf 8 % erhöht wurde.

Zunächst ließen sich ca. 6 g einer Mischung aus Fluoren und Benzofulven eluieren. Anschließend wurden 2,8 g (14 %) der Verbindung 1 eluiert (farbloser Feststoff). Ausbeute 14 % (18 % bzgl. umges. Edukt).
¹H-NMR-Spektrum (CDCl₃): 7,0-8,1 (12,m,Arom.-H), 6,05 (1,t,Ind-H), 4,75 (1,s,Flu-H), 3,42 (2,d,Ind-H2), 1,20 (6,S,CH₃).

### Beispiel 2

### Isopropyliden(9-fluorenyl)-1-inden (1)

Analog Beispiel 1. Die Benzofulven-Lösung wurde jedoch innerhalb 4 h in der Siedehitze zur Lithiumfluoren-Lösung getropft. Anschließend wurde 17 h bei Raumtemperatur gerührt und weitere 4 h zum Rückfluß erhitzt. Aufarbeitung wie in Beispiel 1. Die Säulenchromatograhie lieferte 3,2 g der Verbindung 1 (farbloses Kristallpulver). Ausbeute 17 % (22 % bzgl. umges. Ed.).

### Beispiel 3

### Isopropyliden(9-fluorenyl)-1-inden (1)

Analog Beispiel 1. Die Benzofulven-Lösung wurde jedoch innerhalb 20 min bei -78°C zur Lithiumfluoren-Lösung getropft. Die Mischung wurde in 17 h auf Raumtemperatur erwärmt und 1 h bei Raumtemperatur gerührt. Aufarbeitung wie in Beispiel 1. Die Säulenchromatographie lieferte 9,8 g der Verbindung 1 in Form eines weißen Kristallpulvers. Die Ausbeute betrug 51 %.

### Beispiel 4

### [Isopropyliden(9-fluorenyl)(1-indenyl)]zirkondichlorid (2)

3,2 g (9,9 mmol) des Ligandsystems 1 wurden in 45 ml Diethylether suspendiert und bei Raumtemperatur mit 8,7 ml (21,8 mmol) einer 2,5 M Butyllithiumlösung in Hexan versetzt, wobei die Suspension sich zunächst auflöste und eine hellgelbe Farbe annahm. Nach 21 h Rühren bei Raumtemperatur wurde die entstandene orange Suspension mit 8 ml Hexan versetzt. Der orangefarbene Niederschlag wurde über eine G3-Fritte abgesaugt, mit Hexan gewaschen und 6 h im Öipumpenvakuum bei 50°C getrocknet. Das hellrote Pulver wurde in 30 ml Toluol suspendiert und bei 0°C mit 2,3 g (9,9 mmol) ZrCl₄ versetzt. Nach 30 min Rühren bei Raumtemperatur wurde der entstandene Niederschlag abfiltriert, mit 10 ml Toluol gewaschen und im Ölpumpenvakuum getrocknet. Man erhielt 4,4 g des Komplexes 2 als rotviolettes Pulver. Rohausbeute 92 %. Zur weiteren Reinigung der Verbindung wurde der Rückstand bei -30°C mit einer kleinen Menge Tetrahydrofuran verrührt, um Verunreinigungen zu lösen. Reinausbeute 30 %. Massenspektrum (Direktverdampfung, EI (70 eV), Cl (Isobutan)):
480 M⁺ (korrekter Zerfall).
¹H-NMR-Spektrum (CDCl₃): 6,7-8,1 (12,Arom.-H), 6,50(1,dd,β-Ind-H), 6,07 (1,d,α-Ind-H), 2,92 (3,s,CH₃), 2,60 (3,s,CH₃).

### Beispiel 5

### [Isopropyliden(9-fluorenyl)(1-indenyl)]zirkondichlorid (2)

3,0 g (9,3 mmol) des Ligandsystems 1 wurden analog Beispiel 1 in Diethylether mit Butyllithium umgesetzt und aufgearbeitet. Das Dilithiosalz wurde jedoch bei -78°C zu einer Suspension von 2,0 g (8,4 mmol) ZrCl₄ in 40 ml Toluol gegeben. Die Mischung wurde in 80 min auf 0°C aufgewärmt und noch 30 min bei 0°C gerührt. Der rotviolette Niederschlag wurde abfiltriert, im Ölpumpenvakuum getrocknet und bei -40°C mit 10 ml THF verrührt, um Verunreinigungen zu lösen. Die rotviolette Suspension wurde über eine G3-Fritte filtriert. Man erhielt nach Trocknen im Ölpumpenvakuum 1,6 g des Komplexes 2 als rotviolettes Pulver.
Reinausbeute 40 % (korrektes ¹H-NMR-Spektrum und Massenspektrum).

### Beispiel 6

### Dimethylsilyl(9-fluorenyl)-1-inden (3)

Eine Lösung von 10 g (60,2 mmol) Fluoren in 100 ml Tetrahydrofuran wurde bei Raumtemperatur mit 24 ml (60,2 mmol) einer 2,5 M Butyllithiumlösung in Hexan versetzt und noch 30 min unter Rückfluß erhitzt. Die rote Lösung wurde bei Raumtemperatur langsam zu 23,2 g (180 mmol) Dimethyldichlorsilan, gelöst in 150 ml Tetrahydrofuran, getropft. Nach 16 h Rühren bei Raumtemperatur wurde das Lösemittel abgezogen und das verbleibende gelbliche Öl im Ölpumpenvakuum von überschüssigem Dimethyldichlorsilan befreit. Der gelbliche Feststoff wurde in 100 ml Tetrahydrofuran gelöst und bei Raumtemperatur mit 60,2 mmol Lithiumindenyl, hergestellt aus 7,6 g (60,2 mmol) Inden und 60 mmol Butyllithium in 100 ml Tetrahydrofuran, langsam versetzt. Nach 16 h Rühren bei Raumtemperatur wurde die Mischung auf Eis gegossen, neutral gestellt und mit Diethylether extrahiert. Der nach Abziehen des Lösemittels verbleibende Rückstand wurde an Kieselgel 60 chromatographiert. Mit einem Laufmittelgemisch aus Hexan/Ethylacetat (10:1) wurde zunächst eine Mischung aus Inden und Fluoren eluiert. Danach folgte das Produkt 3 und anschließend die Verbindung Dimethylsilylbis(9-fluoren). Zur weiteren Reinigung wurde die Mischfraktion nochmals mit einem Laufmittelgemisch aus Hexan/Diethylether (40:1) chromatographiert. Insgesamt wurden 1,9 g der reinen Verbindung 3 als weißes Pulver erhalten. Ausbeute 10 %. ¹H-NMR-Spektrum (CDCl₃):
2 Doppelbindungsisomere (A:B=2:1), 7,0-7,9 (Arom.-H), 6,82 (m,Ind-H,A), 6,60 (t,Ind-H,B), 6,32 (dd,Ind-H,A), 4,17 (s,Flu-H,B), 4,05 (s,Flu-H,A), 3,57 (t,Ind-H,A), 3,40 (d, Ind-H,B), mehrere Signale der SiCH₃-Gruppen im Bereich 0 bis -1 ppm.

### Beispiel 7

### [Dimethylsilyl(9-fluorenyl)(1-indenyl)]zirkondichlorid (4)

1,84 g (5,44 mmol) des Ligandsystems 3 wurden in 20 ml Diethylether gelöst und bei Raumtemperatur langsam mit 5,44 ml (13,6 mmol) einer 2,5 M Butyllithiumlösung in Hexan versetzt. Nach 4 h Rühren bei Raumtemperatur wurde die orangegelbe Suspension mit Hexan versetzt und über eine G3-Fritte filtriert. Das orangegelbe Pulver wurde mit 10 ml Hexan gewaschen und lange im Ölpumpenvakuum getrocknet. 1,16 g (5,0 mmol) ZrCl₄ wurden in 20 ml CH₂Cl₂ suspendiert und bei -78°C mit dem Dilithiosalz versetzt. Die Mischung wurde innerhalb 2 h auf Raumtemperatur aufgewärmt und über eine G3-Fritte abfiltriert. Der Feststoff wurde noch mit 5 ml CH₂Cl₂ gewaschen und im Ölpumpenvakuum getrocknet. Man erhielt 1,2 g des Komplexes 4 als rosafarbenes Pulver. Rohausbeute 46 %. Zur vollständigen Reinigung des Komplexes wurde das Rohprodukt in einer kleinen Menge Tetrahydrofuran bei 0°C verrührt, wobei Verunreinigungen in Lösung gingen. Reinausbeute 25 %. Massenspektrum (Direktverdampfung, EI (80 ev)): 496 M+ (korrekter Zerfall, korrektes Isotopenmuster). ¹H-NMR-Spektrum (CDCl₃): 6,9-8,1 (m, 12,Arom.-H), 6,77 (dd,1,β-Ind-H), 5,95 (d,1,α-Ind-H), 1,55 (s,3,CH₃), 1,27 (s,3,CH₃).

### Beispiel 8

### [Dimethylsilyl(9-fluorenyl)(1-indenyl)]hafniumdichlorid (5)

1,50 g (4,43 mmol) des Ligandsystems 3 wurden in 20 ml Diethylether gelöst und analog Beispiel 7 mit Butyllithium umgesetzt und aufgearbeitet. Das Dilithiosalz wurde anschließend analog Beispiel 7 bei -78°C mit 1,42 g (4,43 mmol) HfCl₄ in Methylenchlorid umgesetzt und aufgearbeitet. Man erhielt als Rohprodukt 1,09 g des Komplexes 5 in Form eines rötlichen Pulvers. Rohausbeute 42 %. Zur Reinigung wurde das Pulver bei -10°C mit einer kleinen Menge THF verrührt. Reinausbeute 20 %. Massenspektrum (Direktverdampfung, EI (80 eV)): 587 M⁺ (bzgl. ¹⁸⁰Hf, korrekter Zerfall, korrektes Isotopenmuster). ¹H-NMR-Spektrum (CDCl₃): 7,0-8,1 (m, 12,Arom.-H), 6,61 (dd,1,β-Ind-H), 5,89 (d,1,α-Ind-H), 1,53 (s,3,CH₃), 1,25 (s,3,CH₃).

### Polymerisationsbeispiele:

### Beispiel A:

Ein trockener 16-dm³-Reaktor wurde mit Stickstoff gespült und bei 20°C mit 10 dm³ eines entaromatisierten Benzinschnittes mit dem Siedebereich 100 bis 120°C gefüllt.

Dann wurde der Gasraum des Kessels durch 5-maliges Aufdrücken von 2 bar Ethylen und Entspannen stickstofffrei gespült.

Dann wurden 30 cm³ toluolische Methylaluminoxanlösung (entsprechend 45 mmol Al, Molmasse nach kryoskopischer Bestimmung 750 g/mol) zugegeben.

Unter Rühren wurde der Reaktorinhalt innerhalb von 15 Minuten auf 60°C aufgeheizt und durch Zugabe von Ethylen wurde bei 250 Upm Rührgeschwindigkeit der Gesamtdruck auf 5 bar eingestellt.

Parallel dazu wurden 1,3 mg Dimethylsilyl(9-fluorenyl)(1-indenyl)zirkondichlorid in 20 cm³ toluolischer Methylaluminoxanlösung gelöst und durch 15 minütiges Stehenlassen voraktiviert. Dann wurde die Lösung in den Reaktor gegeben, das Polymerisationssystem wurde auf eine Temperatur von 70°C gebracht und durch entsprechende Kühlung 1 h bei dieser Temperatur gehalten. Der Gesamtdruck wurde während dieser Zeit durch entsprechende Zufuhr von Ethylen bei 5 bar gehalten.

Es wurden 140 g Polyethylen erhalten. Die Viskositätszahl betrug 380 cm³/g.

### Beispiel B:

Ein trockener 16-dm³-Reaktor wurde mit Stickstoff gespült und mit 10 dm³ flüssigem Propylen befüllt.

Dann wurden 30 cm³ toluolische Methylaluminoxanlösung (entsprechend 45 mmol Al, mittlerer Oligomerisierungsgrad n = 16) zugegeben und der Ansatz bei 30°C 15 Minuten gerührt.

Parallel dazu wurden 30,4 mg Isopropyliden(9-fluorenyl)(1-indenyl)zirkondichlorid in 20 cm³ toluolischer Methylaluminoxanlösung (30 mmol Al) gelöst und durch 15 minütiges Stehenlassen voraktiviert.

Die Lösung wurde dann in den Reaktor gegeben, durch Wärmezufuhr wurde auf die Polymerisationstemperatur von 70°C aufgeheizt (4°C/min) und das Polymerisationssystem 1h durch Kühlung bei 70°C gehalten. Gestoppt wurde die Polymerisation durch Zusatz von 20 ml Isopropanol. Das überschüssige Monomere wurde abgegast. Man erhielt 170 g eines hochviskosen, klebrigen Öls.

## Patentansprüche (Patentansprüche für folgende(n) Vertragsstaat(en): AT, BE, DE, FR, GB, IT, NL, SE)

1. Verbindung der Formel I 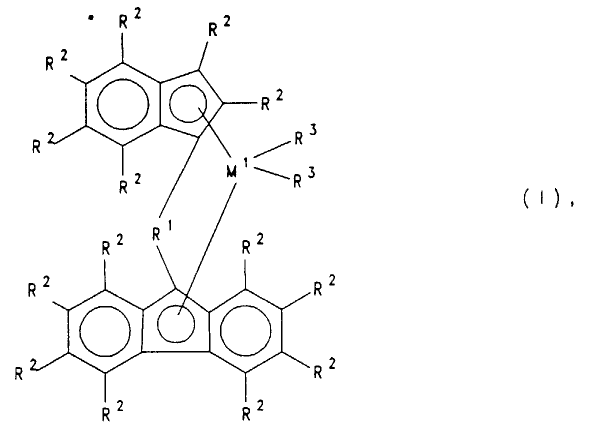 worin M¹ ein Metall aus der Gruppe Titan, Zirkon, Hafnium, Vanadin, Niob und Tantal ist,
R¹ 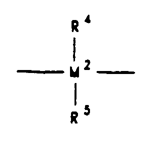 ist, wobei M² Kohlenstoff, Silizium, Germanium oder Zinn bedeutet,
R⁴ und R⁵ gleich oder verschieden sind und ein Wasserstoffatom, ein Halogenatom, eine C₁-C₁₀-Alkylgruppe, eine C₆-C₁₀-Arylgruppe, eine C₂-C₁₀-Alkenylgruppe, eine C₇-C₄₀-Arylalkylgruppe, eine C₈-C₄₀-Arylalkenylgruppe oder eine C₇-C₄₀-Alkylarylgruppe bedeuten, oder R⁴ und R⁵ zusammen mit dem sie verbindenden Atom einen Ring bilden,
die Reste R² gleich oder verschieden sind und Wasserstoff, eine C₁-C₁₀-Alkylgruppe,

2. Verbindung der Formel I nach Anspruch 1, dadurch gekennzeichnet, daß in Formel I M¹ Titan, Zirkon oder Hafnium bedeutet, M² für Kohlenstoff oder Silizium steht, R⁴ und R⁵ gleich sind und Wasserstoff oder eine C₁-C₄-Alkylgruppe bedeuten, R² Wasserstoff oder eine C₁-C₄-Alkylgruppe bedeutet, und die Reste R³ gleich sind und Halogen oder eine C₁-C₄-Alkylgruppe bedeuten.

3. Verbindung der Formel I nach Anspruch 1 oder 2, dadurch gekennzeichnet, daß in Formel I M¹ Zirkon ist, M² für Kohlenstoff oder Silizium steht, R⁴ und R⁵ gleich sind und Methyl oder Ethyl bedeuten, R² Wasserstoff oder eine Methylgruppe bedeutet und die Reste R³ gleich sind und Chlor oder Methyl bedeuten.

4. Isopropyliden(9-fluorenyl)(1-indenyl)zirkondichlorid, Dimethylsilyl(9-fluorenyl)(1 -indenyl)zirkondichlorid.

5. Verfahren zur Herstellung einer Verbindung der Formel I nach einem oder mehreren der Ansprüche 1 bis 4, dadurch gekennzeichnet, daß man
a) eine Verbindung der Formel II 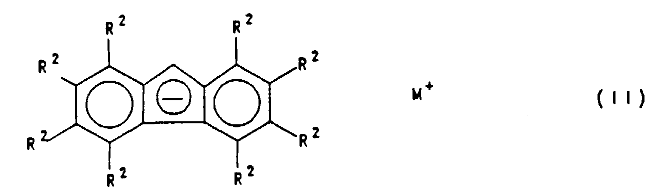 mit einer Verbindung der Formel III
X - R¹ - X (III)
zu einer Verbindung der Formel IV 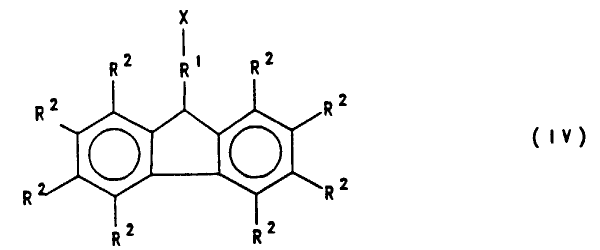 und diese mit einer Verbindung der Formel V 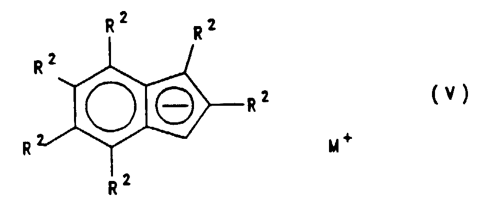 zu einer Verbindung der Formel VI 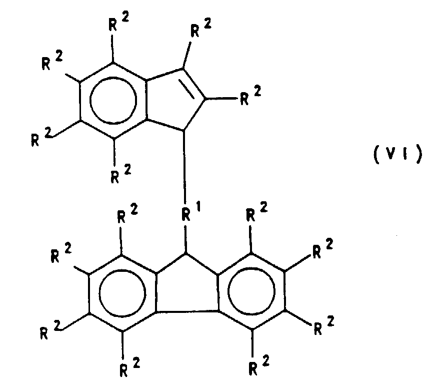 umsetzt, wobei R¹ und R² die für Formel I angegebenen Bedeutungen besitzen, X für eine nucleophile Abgangsgruppe wie Halogen oder O-Tosyl steht und M ein Alkalimetall bedeutet, oder
b) für Verbindungen der Formel I mit 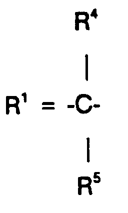 eine Verbindung der Formel VII 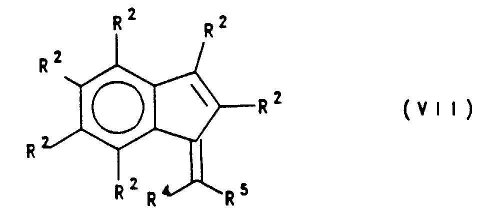 mit einer Verbindung der Formel II zu einer Verbindung der Formel VI umsetzt, wobei die Substituenten die für Formel I genannten Bedeutungen besitzen,
und das unter a) oder b) erhaltene Reaktionsprodukt VI mit einer Verbindung der Formel VIII
M¹X'₄ (VIII),
worin X' für Halogen steht und M¹ die in Formel I genannte Bedeutung hat, umsetzt, und das so erhaltene Reaktionsprodukt gegebenenfalls derivatisiert.

6. Verwendung einer Verbindung der Formel I gemäß einem oder mehreren der Ansprüche 1 bis 4 als Katalysatorkomponente bei der Olefinpolymerisation. eine C₆-C₁₀-Arylgruppe, eine C₂-C₁₀-Alkenylgruppe, eine C₇-C₄₀-Arylalkylgruppe, eine C₈-C₄₀-Arylalkenylgruppe, eine C₇-C₄₀-Alkylarylgruppe oder ein Halogenatom bedeuten, und die Reste R³ gleich oder verschieden und eine C₁-C₁₀-Alkylgruppe, eine C₁-C₁₀-Alkoxygruppe, eine C₆-C₁₀-Arylgruppe, eine C₆-C₁₀-Aryloxygruppe, eine C₂-C₁₀-Alkenylgruppe, eine C₇-C₄₀-Arylalkylgruppe, eine C₈-C₄₀-Arylalkenylgruppe, eine C₇-C₄₀-Alkylarylgruppe, Wasserstoff oder ein Halogenatom bedeuten

7. Polymerisationskatalysator, enthaltend eine Verbindung der Formel I gemäß einen oder mehreren der Ansprüche 1 - 4 und einen Cokatalysator.

8. Verfahren zur Herstellung eines Olefinpolymers in Gegenwart eines Katalysators gemäß Anspruch 7.

9. Verfahren gemäß Anspruch 8, worin Olefine der Formel R^{a}-CH = CH-R^{b} polymerisiert oder copolymerisiert werden, worin R^{a} und R^{b} gleich oder verschieden sind und ein Wasserstoffatom oder einen Alkylrest mit 1 bis 14 C-Atomen bedeuten.

## Patentansprüche (Patentansprüche für folgende(n) Vertragsstaat(en): ES)

1. Verfahren zur Herstellung einer Verbindung der Formel I 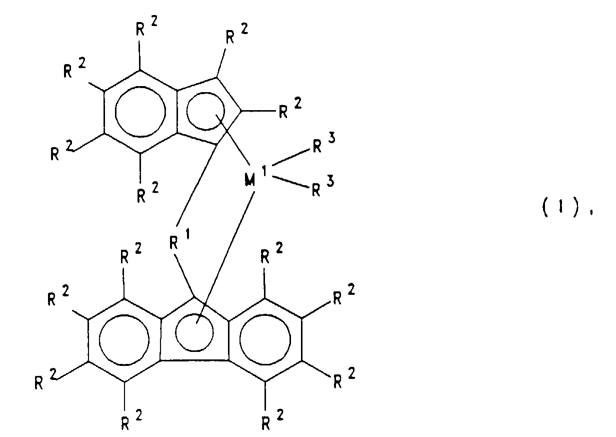 worin M¹ ein Metall aus der Gruppe Titan, Zirkon, Hafnium, Vanadin, Niob und Tantal ist,
R¹ 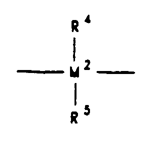 ist, wobei M² Kohlenstoff, Silizium, Germanium oder Zinn bedeutet,
R⁴ und R⁵ gleich oder verschieden sind und ein Wasserstoffatom, ein Halogenatom, eine C₁-C₁₀-Alkylgruppe, eine C₆-C₁₀-Arylgruppe, eine C₂-C₁₀-Alkenylgruppe, eine C₇-C₄₀-Arylalkylgruppe, eine C₈-C₄₀-Arylalkenylgruppe oder eine C₇-C₄₀-Alkylarylgruppe bedeuten, oder R⁴ und R⁵ zusammen mit dem sie verbindenden Atom einen Ring bilden,
die Reste R² gleich oder verschieden sind und Wasserstoff, eine C₁-C₁₀-Alkylgruppe, eine C₆-C₁₀-Arylgruppe, eine C₂-C₁₀-Alkenylgruppe, eine C₇-C₄₀-Arylalkylgruppe, eine C₈-C₄₀-Arylalkenylgruppe, eine C₇-C₄₀-Alkylarylgruppe oder ein Halogenatom bedeuten, und
die Reste R³ gleich oder verschieden und eine C₁-C₁₀-Alkylgruppe, eine C₁-C₁₀-Alkoxygruppe, eine C₆-C₁₀-Arylgruppe, eine C₆-C₁₀-Aryloxygruppe, eine C₂-C₁₀-Alkenylgruppe, eine C₇-C₄₀-Arylalkylgruppe, eine C₈-C₄₀-Arylalkenylgruppe, eine C₇-C₄₀-Alkylarylgruppe, Wasserstoff oder ein Halogenatom bedeuten, dadurch gekennzeichnet, daß man
a) eine Verbindung der Formel II 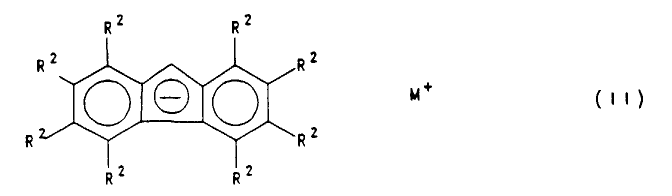 mit einer Verbindung der Formel III
X - R¹- X (III)
zu einer Verbindung der Formel IV 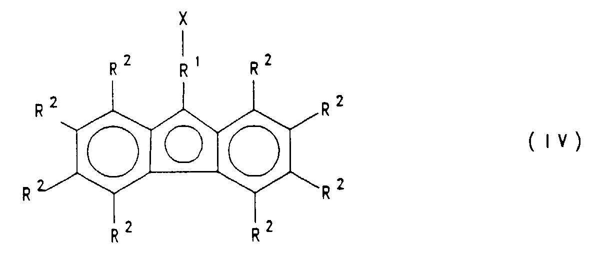 und diese mit einer Verbindung der Formel V 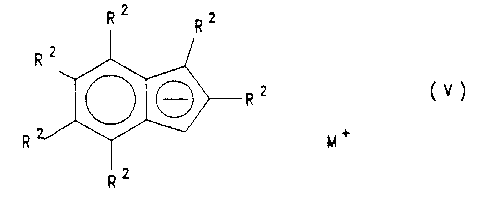 zu einer Verbindung der Formel VI 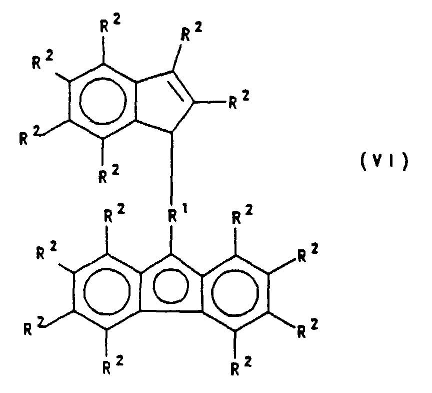 umsetzt, wobei R¹ und R² die für Formel I angegebenen Bedeutungen besitzen, X für eine nucleophile Abgangsgruppe wie Halogen oder O-Tosyl steht und M ein Alkalimetall bedeutet, oder
b) für Verbindungen der Formel I mit 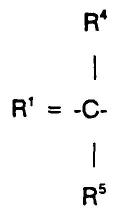 eine Verbindung der Formel VII 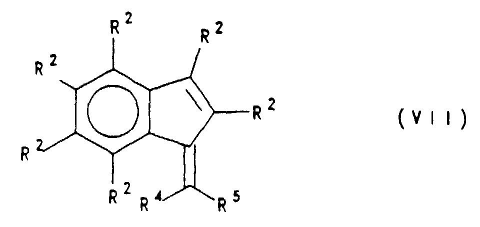 mit einer Verbindung der Formel II zu einer Verbindung der Formel VI umsetzt, wobei die Substituenten die für Formel I genannten Bedeutungen besitzen,
und das unter a) oder b) erhaltene Reaktionsprodukt VI mit einer Verbindung der Formel VIII
M¹X'₄ (VIII),
worin X' für Halogen steht und M¹ die in Formel Igenannte Bedeutung hat, umsetzt, und das so erhaltene Reaktionsprodukt gegebenenfalls derivatisiert.

2. Verfahren nach Anspruch 1, dadurch gekennzeichnet, daß in Formel I M¹ Titan, Zirkon oder Hafnium bedeutet, M² für Kohlenstoff oder Silizium steht, R⁴ und R⁵ gleich sind und Wasserstoff oder eine C₁-C₄-Alkylgruppe bedeuten, R² Wasserstoff oder eine C₁-C₄-Alkylgruppe bedeutet, und die Reste R³ gleich sind und Halogen oder eine C₁-C₄-Alkylgruppe bedeuten.

3. Verfahren nach Anspruch 1 oder 2, dadurch gekennzeichnet, daß in Formel I M¹ Zirkon ist, M² für Kohlenstoff oder Silizium steht, R⁴ und R⁵ gleich sind und Methyl oder Ethyl bedeuten, R² Wasserstoff oder eine Methylgruppe bedeutet und die Reste R³ gleich sind und Chlor oder Methyl bedeuten.

4. Verfahren gemäß einem oder mehreren der Ansprüche 1 bis 3, dadurch gekennzeichnet, daß Isopropyliden(9-fluorenyl)(1-indenyl)zirkondichlorid oder Dimethylsilyl(9-fluorenyl)-(1-indenyl)zirkondichlorid hergestellt wird.

5. Verwendung einer Verbindung der Formel I gemäß einem oder mehreren der Ansprüche 1 bis 4 als Katalysatorkomponente bei der Olefinpolymerisation.

6. Verfahren zur Herstellung eines Olefinpolymers in Gegenwart eines Katalysators enthaltend eine Verbindung der Formel I herstellbar nach einem der Ansprüche 1-4 und ein Cokatalysator.

7. Verfahren nach Anspruch 6, worin Olefine der Formel R^{a}-CH=CH-R^{b} polymerisiert oder copolymerisiert werden, worin R^{a} und R^{b} gleich oder verschieden sind und ein Wasserstoffatom oder ein Alkylrest mit 1 bis 14 C-Atomen bedeuten.

## Claims (Claims for the following Contracting State(s): AT, BE, DE, FR, GB, IT, NL, SE)

1. A compound of the formula I 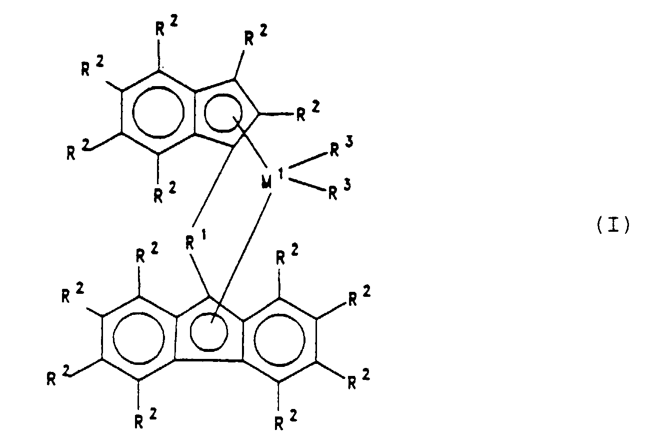 in which M¹ is a metal from the group consisting of titanium, zirconium, hafnium, vanadium, niobium and tantalum,
R¹ is 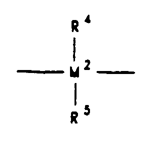 in which M² is carbon, silicon, germanium or tin,
R⁴ and R⁵ are identical or different and are a hydrogen atom, a halogen atom, a C₁-C₁₀-alkyl group, a C₆-C₁₀-aryl group, a C₂-C₁₀-alkenyl group, a C₇-C₄₀-arylalkyl group, a C₈-C₄₀-arylalkenyl group or a C₇-C₄₀-alkylaryl group, or R⁴ and R⁵, together with the atom joining them, form a ring,
the radicals R² are identical or different and are hydrogen, a C₁-C₁₀-alkyl group, a C₆-C₁₀-aryl group, a C₂-C₁₀-alkenyl group, a C₇-C₄₀-arylalkyl group, a C₈-C₄₀-arylalkenyl group, a C₇-C₄₀-alkylaryl group or a halogen atom and the radicals R³ are identical or different and are a C₁-C₁₀-alkyl group, a C₁-C₁₀-alkoxy group, a C₆-C₁₀-aryl group, a C₆-C₁₀-aryloxy group, a C₂-C₁₀-alkenyl group, a C₇-C₄₀-arylalkyl group, a C₈-C₄₀-arylalkenyl group, a C₇-C₄₀-alkylaryl group, hydrogen or a halogen atom.

2. A compound of the formula I as claimed in claim 1, in which, in formula I, M¹ is titanium, zirconium or hafnium, M² is carbon or silicon, R⁴ and R⁵ are identical and are hydrogen or a C₁-C₄-alkyl group, R² is hydrogen or a C₁-C₄-alkyl group and the radicals R³ are identical and are halogen or a C₁-C₄-alkyl group.

3. A compound of the formula I as claimed in claim 1 or 2, in which, in formula I, M¹ is zirconium, M² is carbon or silicon, R⁴ and R⁵ are identical and are methyl or ethyl, R² is hydrogen or a methyl group and the radicals R³ are identical and are chlorine or methyl.

4. Isopropylidene (9-fluorenyl) (1-indenyl) zirconium dichloride or dimethylsilyl(9-fluorenyl) (1-indenyl)zirconium dichloride.

5. A process for the preparation of a compound of the formula I as claimed in one or more of claims 1 to 4, which comprises
a) reacting a compound of the formula II 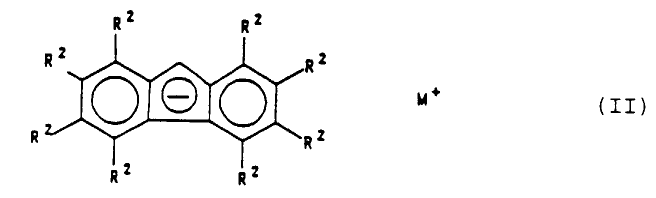 with a compound of the formula III
X-R¹-X (III)
to give a compound of the formula IV 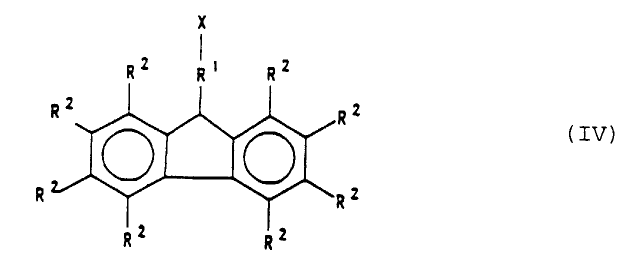 and reacting this with a compound of the formula V 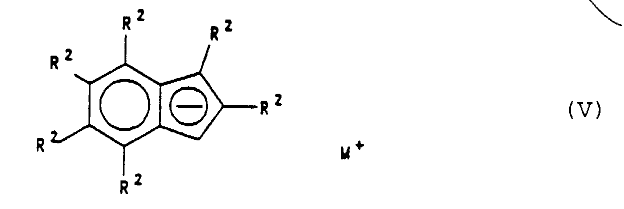 to give a compound of the formula VI 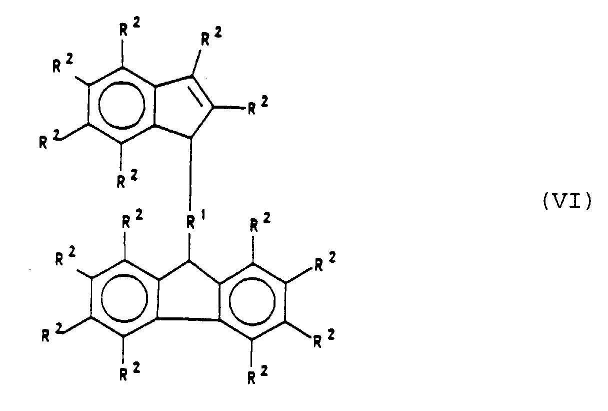 in which R¹ and R² have the meanings given for formula I, X is a nucleophilic leaving group, such as halogen or O-tosyl, and M is an alkali metal, or
b) for a compound of the formula I where 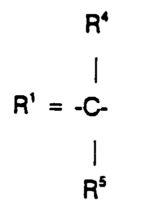 reacting a compound of the formula VII 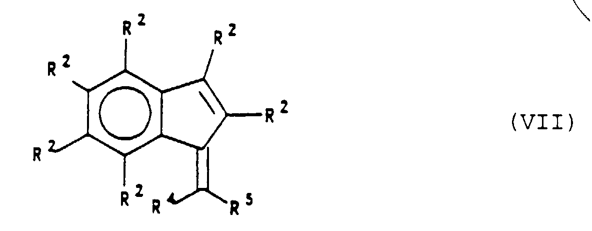 with a compound of the formula II to give a compound of the formula VI, in which the substituents have the meanings given for formula I,
and reacting the reaction product VI obtained under a) or b) with a compound of the formula VIII
M¹X'₄ (VIII)
in which X' is halogen and M¹ has the meaning given in formula I, and if appropriate forming derivatives of the reaction product thus obtained.

6. The use of a compound of the formula I as claimed in one or more of claims 1 to 4 as a catalyst component in olefin polymerization.

7. A polymerization catalyst comprising a compound of the formula I as claimed in one or more of claims 1 - 4 and a cocatalyst.

8. A process for the preparation of an olefin polymer in the presence of a catalyst as claimed in claim 7.

9. A process as claimed in claim 8, in which olefins of the formula R^{a}-CH=CH-R^{b} are polymerized or copolymerized, in which R^{a} and R^{b} are identical or different and are a hydrogen atom or an alkyl radical having 1 to 14 carbon atoms.

## Claims (Claims for the following Contracting State(s): ES)

1. A process for the preparation of a compound of the formula I 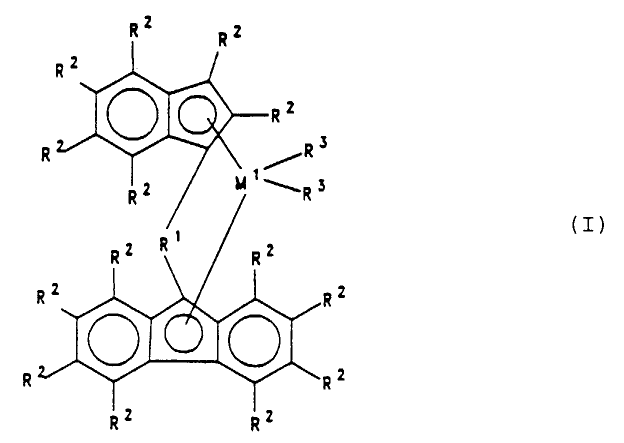 in which M¹ is a metal from the group consisting of titanium, zirconium, hafnium, vanadium, niobium and tantalum,
R¹ is 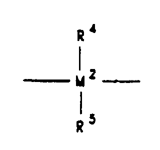 in which M² is carbon, silicon, germanium or tin,
R⁴ and R⁵ are identical or different and are a hydrogen atom, a halogen atom, a C₁-C₁₀-alkyl group, a C₆-C₁₀-aryl group, a C₂-C₁₀-alkenyl group, a C₇-C₄₀-arylalkyl group, a C₈-C₄₀-arylalkenyl group or a C₇-C₄₀-alkylaryl group, or R⁴ and R⁵, together with the atom joining them, form a ring,
the radicals R² are identical or different and are hydrogen, a C₁-C₁₀-alkyl group, a C₆-C₁₀-aryl group, a C₂-C₁₀-alkenyl group, a C₇-C₄₀-arylalkyl group, a C₈-C₄₀-arylalkenyl group, a C₇-C₄₀-alkylaryl group or a halogen atom and
the radicals R³ are identical or different and are a C₁-C₁₀-alkyl group, a C₁-C₁₀-alkoxy group, a C₆-C₁₀-aryl group, a C₆-C₁₀-aryloxy group, a C₂-C₁₀-alkenyl group, a C₇-C₄₀-arylalkyl group, a C₈-C₄₀-arylalkenyl group, a C₇-C₄₀-alkylaryl group, hydrogen or a halogen atom, which comprises
a) reacting a compound of the formula II 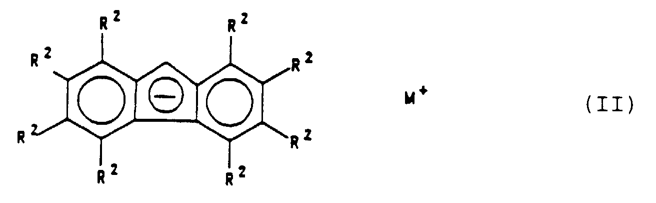 with a compound of the formula III
X-R¹-X (III)
to give a compound of the formula IV 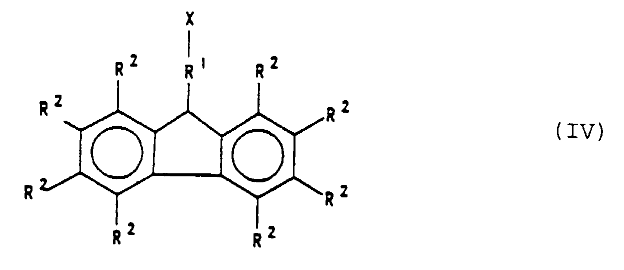 and reacting this with a compound of the formula V 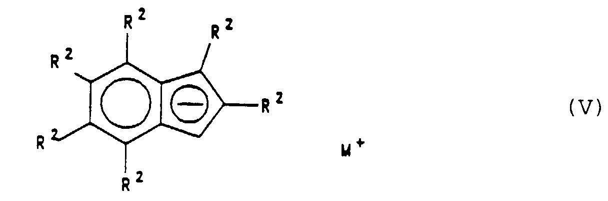 to give a compound of the formula VI 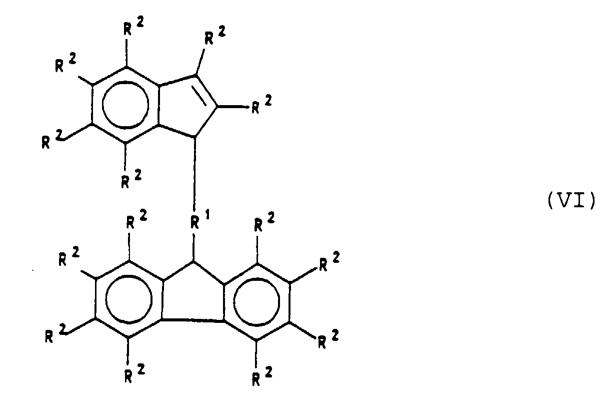 in which R¹ and R² have the meanings given for formula I, X is a nucleophilic leaving group, such as halogen or O-tosyl, and M is an alkali metal, or
b) for a compound of the formula I where 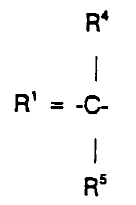 reacting a compound of the formula VII 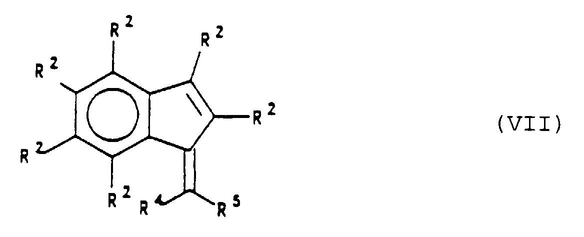 with a compound of the formula II to give a compound of the formula VI, in which the substituents have the meanings given for formula I,
and reacting the reaction product VI obtained under a) or b) with a compound of the formula VIII
M¹X'₄ (VIII)
in which X' is halogen and M¹ has the meaning given in formula I, and if appropriate forming derivatives of the reaction product thus obtained.

2. A process I as claimed in claim 1, in which, in formula I, M¹ is titanium, zirconium or hafnium, M² is carbon or silicon, R⁴ and R⁵ are identical and are hydrogen or a C₁-C₄-alkyl group, R² is hydrogen or a C₁-C₄-alkyl group and the radicals R³ are identical and are halogen or a C₁-C₄-alkyl group.

3. A process as claimed in claim 1 or 2, in which, in formula I, M¹ is zirconium, M² is carbon or silicon, R⁴ and R⁵ are identical and are methyl or ethyl, R² is hydrogen or a methyl group and the radicals R³ are identical and are chlorine or methyl.

4. A process as claimed in one or more of claims 1 to 3, wherein isopropylidene(9-fluorenyl) (1-indenyl)zirconium dichloride or dimethylsilyl (9-fluorenyl)(1-indenyl) zirconium dichloride is prepared.

5. The use of a compound of the formula I as claimed in one or more of claims 1 to 4 as a catalyst component in olefin polymerization.

6. A process for the preparation of an olefin polymer in the presence of a catalyst comprising a compound of the formula I, which can be prepared as claimed in any of claims 1 - 4, and a cocatalyst.

7. A process as claimed in claim 6, in which olefins of the formula R^{a}-CH=CH-R^{b} are polymerized or copolymerized, in which R^{a} and R^{b} are identical or different and are a hydrogen atom or an alkyl radical having 1 to 14 carbon atoms.

## Revendications (Revendications pour l'(les) Etat(s) contractant(s) suivant(s): AT, BE, DE, FR, GB, IT, NL, SE)

1. Composé de la formule I : 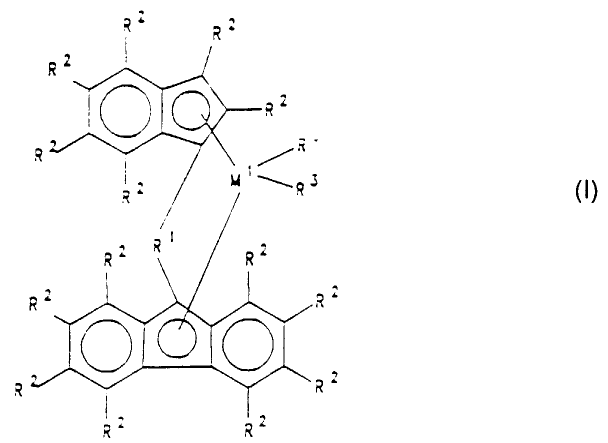 dans laquelle M¹ représente un métal choisi dans le groupe formé par le titane, le zirconium, l'hafnium, le vanadium, le niobium et le tantale,
R¹ représente un radical 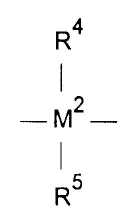 où M² représente le carbone, le silicium, le germanium ou l'étain,
R⁴ et R⁵ sont identiques ou différents et représentent chacun un atome d'hydrogène, un atome d'halogène, un radical alkyle en C₁ à C₁₀, un radical aryle en C₆ à C₁₀, un radical alcényle en C₂ à C₁₀, un radical arylalkyle en C₇ à C₄₀, un radical arylalcényle en C₈ à C₄₀, ou un radical alkylaryle en C₇ à C₄₀, ou bien R⁴ et R⁵ forment un cycle avec l'atome qui les relie,
les symboles R² sont identiques ou différents et représentent chacun un atome d'hydrogène, un groupe alkyle en C₁ à C₁₀, un groupe aryle en C₆ à C₁₀, un groupe alcényle en C₂ à C₁₀, un groupe arylalkyle en C₇ à C₄₀, un groupe arylalcényle en C₈ à C₄₀, un groupe alkylaryle en C₇ à C₄₀ ou un atome d'halogène, et
les symboles R³ sont identiques ou différents et représentent chacun un groupe alkyle en C₁ à C₁₀, un groupe alcoxy en C₁ à C₁₀, un groupe aryle en C₆ à C₁₀, un groupe aryloxy en C₆ à C₁₀, un groupe alcényle en C₂ à C₁₀, un groupe arylalkyle en C₇ à C₄₀, un groupe arylalcényle en C₈ à C₄₀, un groupe alkylaryle en C₇ à C₄₀, un atome d'hydrogène, ou un atome d'halogène.

2. Composé de la formule I suivant la revendication 1, caractérisé en ce que dans la formule I, M¹ représente le zirconium ou l'hafnium, M² représente le carbone ou le silicium, R⁴ et R⁵ sont identiques et représentent chacun un atome d'hydrogène ou un radical alkyle en C₁ à C₄, R² représente un atome d'hydrogène ou un radical alkyle en C₁ à C4 et les restes R³ sont identiques ou différents et représentent des atomes d'halogènes ou des radicaux alkyle en C₁ à C₄.

3. Composé de la formule I suivant la revendication 1 ou 2, caractérisé en ce que dans la formule I, M¹ représente le zirconium, M² représente le carbone ou le silicium, R⁴ et R⁵ sont identiques ou différents et représentent chacun un radical méthyle ou éthyle, R² représente un atome d'hydrogène ou le radical méthyle et les restes R³ sont identiques et représentent des atomes de chlore ou des radicaux méthyle.

4. Dichlorure d'isopropylidène-(9-fluorényl)(1-indényl)-zirconium, dichlorure de diméthylsilyl-(9-fluorényl)(1-indényl)zirconium.

5. Procédé de préparation d'un composé de la formule I suivant une ou plusieurs des revendications 1 à 4, caractérisé en ce que l'on fait réagir
a) un composé de la formule II : 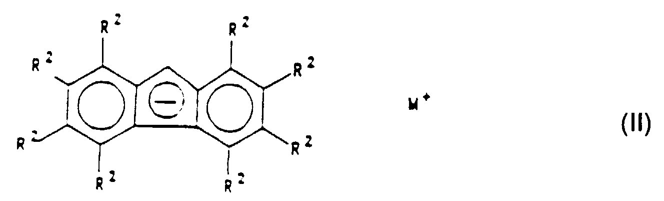 avec un composé de la formule III :
X - R¹ - X (III)
de manière à obtenir un composé de la formule IV: 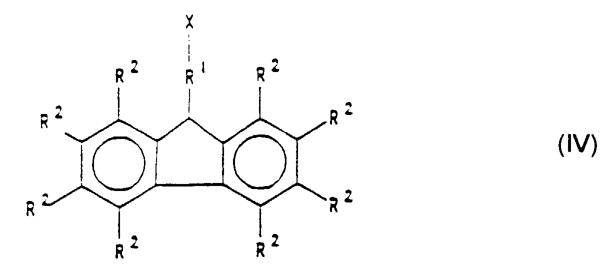 et on fait réagir celui-ci avec un composé de la formule V : 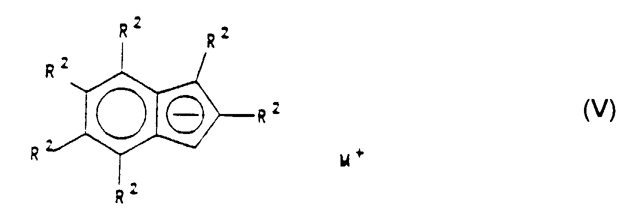 de manière à obtenir un composé de la formule VI : 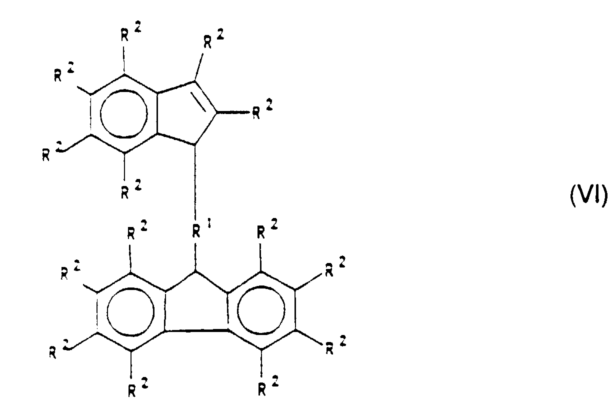 où R¹ et R² possèdent les significations qui leur ont été attribuées à propos de la définition de la formule I, X représente un groupe sortant nucléophile, comme un atome d'halogène ou le radical O-tosyle et M représente un métal alcalin, ou bien
b) pour des composés de la formule I avec R¹ = 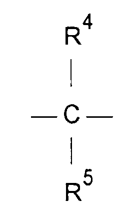 on fait réagir un composé de la formule VII : 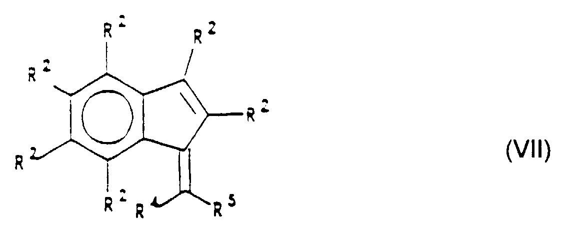 avec un composé de la formule Il de manière à obtenir un composé de la formule VI, où les substituants possèdent les significations qui leur ont été attribuées à propos de la définition de la formule 1,
et on fait réagir le produit de réaction VI obtenu sous a) ou b) avec un composé de la formule VIII :
M¹X'₄ (VIII)
où X' représente un atome d'halogène et M¹ possède les significations qui lui ont été attribuées à propos de la définition de la formule I et on dérivatise éventuellement le produit de réaction ainsi obtenu.

6. Utilisation d'un composé de la formule I suivant une ou plusieurs des revendications 1 à 4, à titre de composant de catalyseur pour la polymérisation d'oléfines.

7. Catalyseur de polymérisation qui contient un composé de la formule I suivant une ou plusieurs des revendications 1 à 4 et un cocatalyseur.

8. Procédé de préparation d'un polymère d'oléfine en présence d'un catalyseur suivant la revendication 7.

9. Procédé suivant la revendication 8, caractérisé en ce que l'on polymérise ou copolymérise des oléfines de la formule R^{a}-CH=CH-R^{b} dans laquelle R^{a} et R^{b} sont identiques ou différents et représentent chaucn un atome d'hydrogène ou un reste alkyle comportant de 1 à 14 atomes de carbone.

## Revendications (Revendications pour l'(les) Etat(s) contractant(s) suivant(s): ES)

1. Procédé de préparation d'un composé de la formule I : 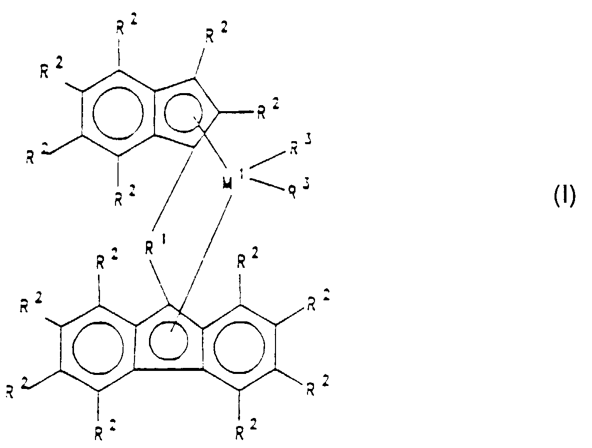 dans laquelle M¹ représente un métal choisi dans le groupe formé par le titane, le zirconium, l'hafnium, le vanadium, le niobium et le tantale,
R¹ représente un radical 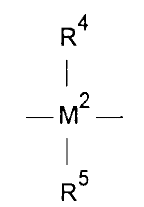 où M² représente le carbone, le silicium, le germanium ou l'étain,
R⁴ et R⁵ sont identiques ou différents et représentent chacun un atome d'hydrogène, un atome d'halogène, un radical alkyle en C₁ à C₁₀, un radical aryle en C₆ à C₁₀, un radical alcényle en C₂ à C₁₀, un radical arylalkyle en C₇ à C₄₀, un radical arylalcényle en C₈ à C₄₀, ou un radical alkylaryle en C₇ à C₄₀, ou bien R⁴ et R⁵ forment un cycle avec l'atome qui les relie,
les symboles R² sont identiques ou différents et représentent chacun un atome d'hydrogène, un groupe alkyle en C₁ à C₁₀, un groupe aryle en C₆ à C₁₀, un groupe alcényle en C₂ à C₁₀, un groupe arylalkyle en C₇ à C₄₀, un groupe arylalcényle en C₈ à C₄₀, un groupe alkylaryle en C₇ à C₄₀ ou un atome d'halogène, et
les symboles R³ sont identiques ou différents et représentent chacun un groupe alkyle en C₁ à C₁₀, un groupe alcoxy en C₁ à C₁₀, un groupe aryle en C₆ à C₁₀, un groupe aryloxy en C₆ à C₁₀, un groupe alcényle en C₂ à C₁₀, un groupe arylalkyle en C₇ à C₄₀, un groupe arylalcényle en C₈ à C₄₀, un groupe alkylaryle en C₇ à C₄₀, un atome d'hydrogène, ou un atome d'halogène, caractérisé en ce que l'on fait réagir
a) un composé de la formule II : 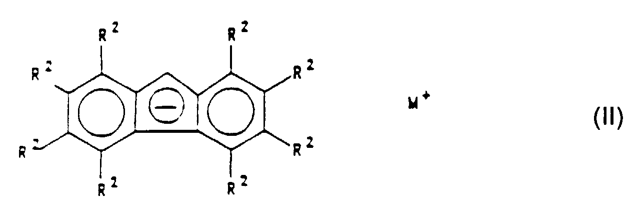 avec un composé de la formule III :
X - R¹ - X (III)
de manière à obtenir un composé de la formule IV: 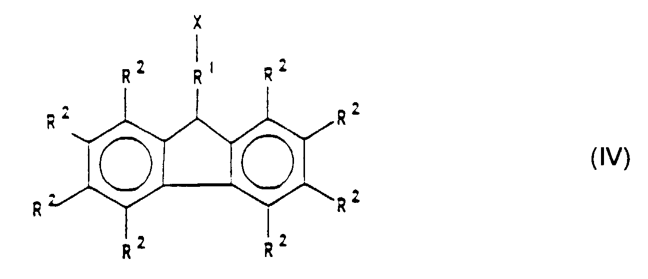 et on fait réagir celui-ci avec un composé de la formule V: 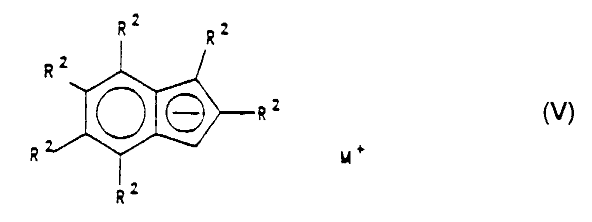 de manière à obtenir un composé de la formule VI : 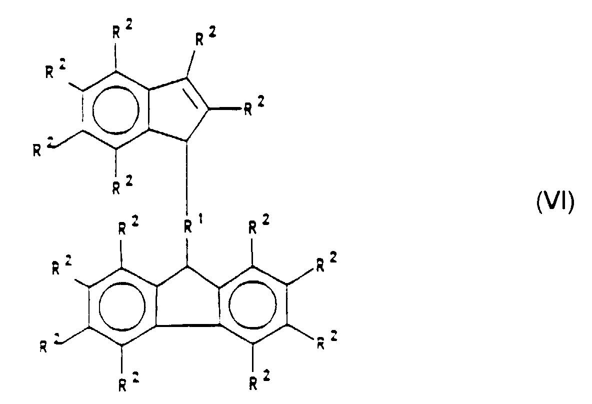 où R¹ et R² possèdent les significations qui leur ont été attribuées à propos de la définition de la formule I, X représente un groupe sortant nucléophile, comme un atome d'halogène ou le radical O-tosyle et M représente un métal alcalin, ou bien
b) pour des composés de la formule I avec R¹ = 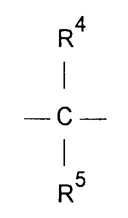 on fait réagir un composé de la formule VII : 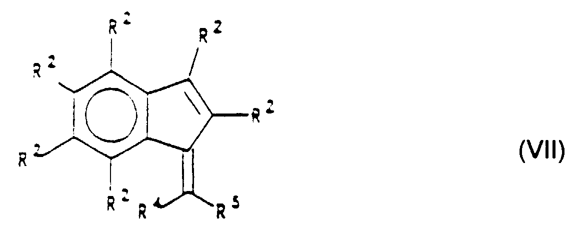 avec un composé de la formule Il de manière à obtenir un composé de la formule VI, où les substituants possèdent les significations qui leur ont été attribuées à propos de la définition de la formule I,
et on fait réagir le produit de réaction VI obtenu sous a) ou b) avec un composé de la formule VIII :
M¹X'₄ (VIII)
où X' représente un atome d'halogène et M¹ possède les significations qui lui ont été attribuées à propos de la définition de la formule I et on dérivatise éventuellement le produit de réaction ainsi obtenu.

2. Procédé suivant la revendication 1, caractérisé en ce que dans la formule I M¹ représente le zirconium ou l'hafnium, M² représente le carbone ou le silicium, R⁴ et R⁵ sont identiques et représentent chacun un atome d'hydrogène ou un radical alkyle en C₁ à C₄, R² représente un atome d'hydrogène ou un radical alkyle en C₁ à C₄ et les restes R³ sont identiques ou différents et représentent des atomes d'halogènes ou des radicaux alkyle en C₁ à C₄.

3. Procédé suivant la revendication 1 ou 2, caractérisé en ce que dans la formule M¹ représente le zirconium, M² représente le carbone ou le silicium, R⁴ et R⁵ sont identiques ou différents et représentent chacun un radical méthyle ou éthyle, R² représente un atome d'hydrogène ou le radical méthyle et les restes R³ sont identiques et représentent des atomes de chlore ou des radicaux méthyle.

4. Procédé suivant une ou plusieurs des revendications 1 à 3, caractérisé en ce que l'on prépare le dichlorure d'isopropylidène-(9-fluorényl)(1-indényl)-zirconium ou le dichlorure de diméthylsilyl-(9-fluorényl)(1-indényl)zirconium.

5. Utilisation d'un composé de la formule I suivant une ou plusieurs des revendications 1 à 4, à titre de composant de catalyseur pour la polymérisation d'oléfines.

6. Procédé de préparation d'un polymère d'oléfine en présence d'un catalyseur contenant un composé de la formule I que l'on peut préparer suivant l'une quelconque des revendications 1 à 4 et un cocatalyseur.

7. Procédé suivant la revendication 6, caractérisé en ce que l'on polymérise ou copolymérise des oléfines de la formule R^{a}-CH=CH-R^{b} dans laquelle R^{a} et R^{b} sont identiques ou différents et représentent chacun un atome d'hydrogène ou un radical alkyle comportant de 1 à 14 atomes de carbone.
